(19) Europäisches Patentamt
European Patent Office
Office européen des brevets

(11) Publication number: **0 166 726**
**B1**

(12) # EUROPEAN PATENT SPECIFICATION

(45) Date of publication of patent specification: **17.11.88**

(51) Int. Cl.$^4$: **C 07 D 471/04**, A 61 K 31/505 // C07C69/82, (C07D471/04, 239:00, 211:00)

(21) Application number: **84900591.3**

(22) Date of filing: **27.12.83**

(86) International application number: **PCT/US83/02065**

(87) International publication number: **WO 85/02844 04.07.85 Gazette 85/15**

(54) **10-ETHYL-8,10-DIDEAZAMINOPTERINS.**

(43) Date of publication of application: **08.01.86 Bulletin 86/02**

(45) Publication of the grant of the patent: **17.11.88 Bulletin 88/46**

(84) Designated Contracting States: **BE FR**

(56) References cited:
**Chemical Abstracts, vol. 100, no. 9, 27 February 1984 (Columbus, Ohio, US) J.I. de Graw: "Synthesis and activity of 8,10-dideazaminopterin" see page 636, abstract no. 68669p, & Chem. Biol. Pteridines, Proc. Int. Symp. Pteridines folic Acid Deriv.: Chem., Biol. Clin. Aspects, 7th 1982 (Pub. 1983) 457-61 Chem. Biol. clin. Aspects, 7th 1982 (Publ. 1983), p. 457-461**

(73) Proprietor: **SRI INTERNATIONAL**
**333 Ravenswood Avenue**
**Menlo Park, California 94025 (US)**

(72) Inventor: **DeGRAW, Joseph I.**
**880 Hanover**
**Sunnyvale, CA 94087 (US)**
Inventor: **KELLY, Lawrence F.**
**Australian National University Box 4, P.O.**
**Canberra ACT 2600 (AU)**
Inventor: **SIROTNAK, Francis M.**
**Memorial Sloan-Kettering Cancer Center**
**1275 York Avenue New York, NY 10021 (US)**

(74) Representative: **Rinuy, Santarelli**
**14, avenue de la Grande Armée**
**F-75017 Paris (FR)**

**EP 0 166 726 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

REFERENCE TO GOVERNMENT GRANT

The invention described herein was made in the course of work under grants from the National Institute of Health.

TECHNICAL FIELD

The invention is in the field of pterin chemistry and cancer therapy. More specifically it concerns 10-ethyl-8,10-dideazaminopterin and its use as antineoplastic agent.

BACKGROUND ART

Aminopterin and its N-10-methyl derivative, methotrexate, have long been recognized as powerful antineoplastic agents. Methotrexate has enjoyed some thirty years of acceptance as a clinically useful anticancer drug. The drugs are antimetabolites inhibiting dihydrofolate reductase (DHFR). They affect both neoplastic and normal host tissue.

J. I. DeGraw and F. M. Sirotnak, *Cancer Treatment Reports, 62,* 1047 (1978), British Patent 1,534,238, dated November 29, 1978 and U.S. patent 4,393,064, filed 17 September 1979 report that 10-deazaminopterin exhibited more potent antitumor activity than aminopterin and methotrexate in experimental tumors in mice. Specifically, it was found to have a wider spectrum of activity, better penetration of tumor tissue and more favorable distribution and kinetic parameters for tumor versus normal tissue. The cited U.S. patent also teaches that antitumor effect and the spectrum of activity of 10-deazaminopterin were enhanced by incorporation of alkyl groups of short chain length at the 10 carbon atom.

Alterations of the pteridine ring of folic acid have also been investigated. The synthesis and antifolate activity of 8-deazafolic acid was reported by J. I. DeGraw, R. L. Kisliuk, Y. Gaumont and C. M. Baugh *J Med Che,* (1974) *17*:470 and 8,10-dideazafolic acid was reported in J. I. Degraw, R. L. Kisliuk, V. H. Brown and Y. Gaumont, *Chem Biol Pteridines* (1979) *6*:229. Both of these compounds were active antifolates, but did not significantly affect DHFR. A. Srinivasan and A. D. Broom, *J. Org Chem* (1981) *46*:1777 reported the preparation of 8-deazaminopterin and 8-deazamethotrexate, but did not report biological activity for the compounds.

More recently, it was disclosed that 8,10-dideazaminopterin exhibited biological activity (DeGraw, J. I., et al, *J Hetero Chem,* (1982) *19*:1587. A poster session at the *International Symposium on Pteridine and Folid Acid Analogs,* Scotland in September 1982 disclosed preparation of the n-propyl analog.

Moreover, in "Chem. Bio. Clin. Aspects, 7th 1982 (Pub 1983), pages 457—61", the synthesis the 10-methyl, 10-propyl and 10-amyl derivatives of 8,10-dideazaminopterin is described with the indication that this 10-propyl analog has a decreased potency as compared with the unsubstituted compound.

It has now been found that 10-ethyl-8,10-dideazminopterin has a surprisingly potent anti-neoplastic activity as compared with the unsubstituted and n-propyl or methyl analogs.

DISCLOSURE OF THE INVENTION

The compounds of the invention are 10-ethyl-8,10-dideazaminopterin of the formula:

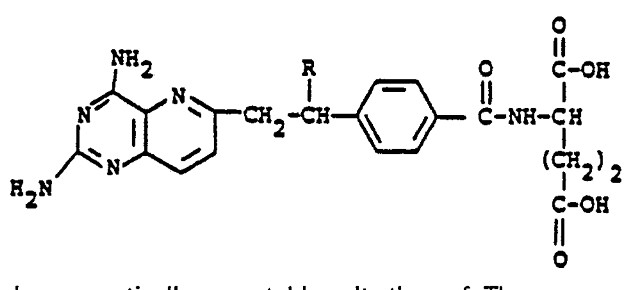

where R is ethyl and pharmaceutically acceptable salts thereof. These compounds have exceptional anti-neoplastic activity.

Antineoplastic compositions containing the compounds comprise a therapeutically effective amount of one or more of the compounds in combination with a pharmaceutically acceptable carrier. Living animals, including humans, are treated for cancer by administering therapeutically effective amounts of such compositions, typically in a unit parenteral dosage form, to the animal.

MODES FOR CARRYING OUT THE INVENTION

The pharmaceutically acceptable salts of this compound include acid addition salts and carboxylate salts. These salts are "pharmaceutically acceptable" in that they are nontoxic and functionally equivalent to the base compound. Acid addition salts are formed by reacting one or more of the free $NH_2$ groups of the ethyl 8,10-dideazaminopterin with an appropriate acid. Suitable acids include inorganic acids, for example, hydrochloric, hydrobromic, nitric, sulfuric or phosphoric acid; organic carboxylic acids such as glycolic,

hydroxymaleic, malic, tartaric, citric, salicylic, o-acetyloxybenzoic, nicotinic and isonicotinic acid; and organic sulphonic acids such as methanesulfonic, ethanesulfonic, 2-hydroxyethane sulfonic, p-toluenesulfonic or naphthalene-2-sulfonic acid.

Carboxylate salts are formed by reacting a suitable base with one or more of the free carboxylic groups of the ethyl 8,10-dideazaminopterin. Suitable bases include alkali metal or alkaline earth metal hydroxide, potassium hydroxide, calcium hydroxide, magnesium hydroxide and corresponding carbonates; and nitrogen bases such as ammonia and alkylamines such as trimethylamine, and triethylamine.

The compounds of this invention may be synthesized by the following route:

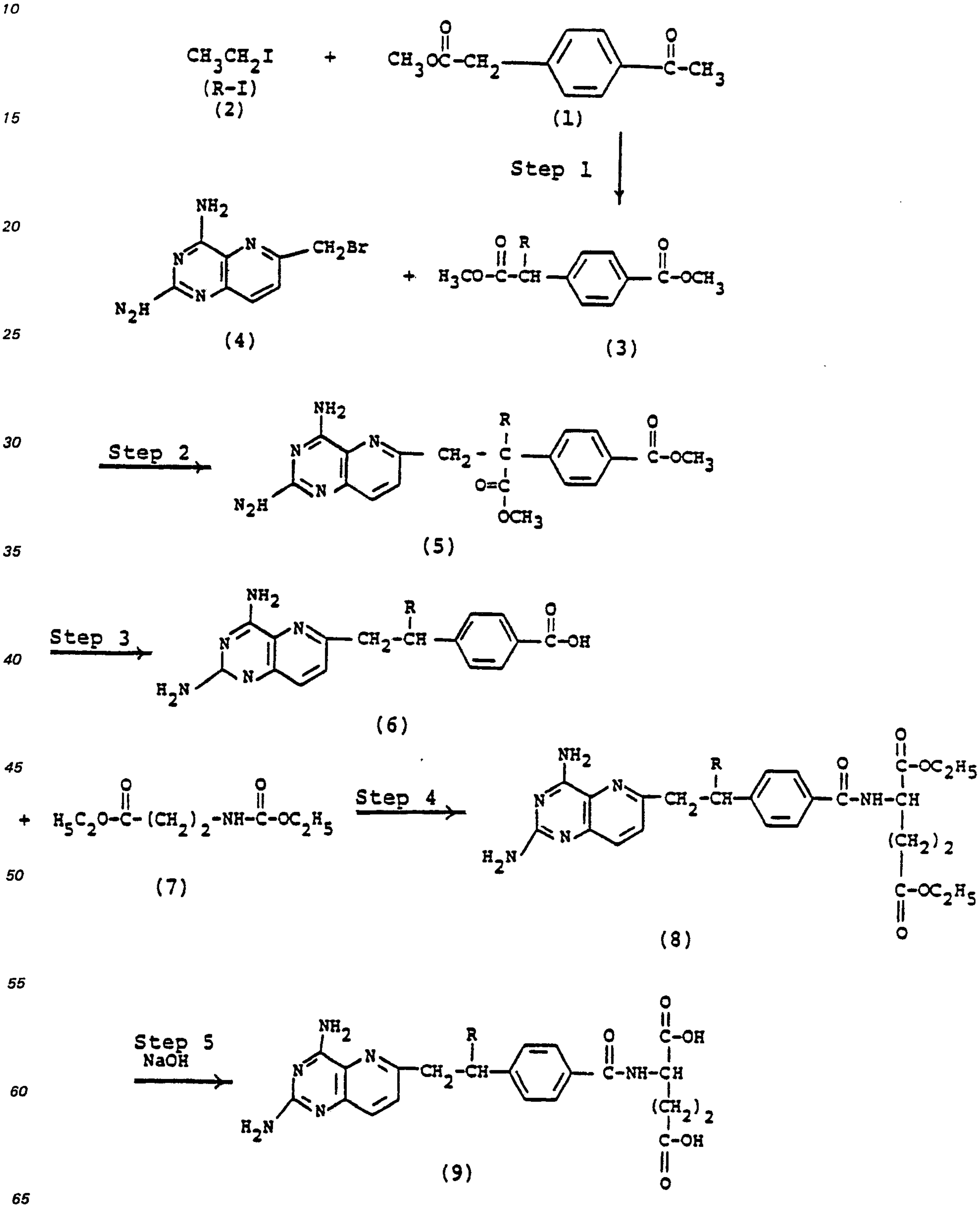

The reaction scheme as shown uses the synthesis of the 10-ethyl derivative.

In step 1, the alkyl iodide (2) is reacted with a homoterephthalate ester (1). Any common homoterephthalate ester may be used, and the intermediate anion in the alkylation is generated by suitable base, such as alkali hydride, hydroxide or alkoxide in a polar aprotic solvent such as DMF or THF. The reaction is quenched with acid, and the intermediate α-alkyl homoterephthalate isolated.

In step 2 the bromomethyl compound (4) is used to alkylate the anion of the dimethyl α-alkyl homoterephthalate. Any common ester of the acid portion of (3) may be used and the anion may be generated by alkali hydrides, alkoxides, hydroxides and other bases obvious to one skilled in the art. A suitable polar aprotic solvent such as dimethyl formamide is employed. The 10-carboxylate compound (5) so obtained is treated in dimethyl sulfoxide or hexamethyl phosphoramide solvent with an alkali metal halide or cyanide to cause cleavage of the 10-carboxylate ester with subsequent decarboxylation of the 10-carboxylic acid to afford a 2,4-diaminopteroic acid intermediate (6). The useful temperature range for the process is 100°—200°C, preferably about 170°—180°C. The pteroic acid compound (6) is then coupled with diethyl L-glutamate (7). Activation of the carboxylate moiety of (6) is achieved via the mixed anhydride as generated in the presence of isobutyl chloroformate and triethyl amine. Other chloroformate esters along with other organic bases would be suitable for this purpose. The pteroyl glutamate diester (8) so obtained is hydrolyzed via treatment with an alkali hydroxide or carbonate in aqueous alcoholic medium, preferably aqueous 2-methoxy-ethanol to give the 10-alkyl-8,10-dideazaminopterin (9).

MODE OF ADMINISTRATION

The physiological properties of 8,10-dideazaminopterins of the invention are similar to methotrexate. Accordingly they may be formulated for use as antineoplastic agents in a manner similar to methotrexate. Such formulations will comprise the 8,10-dideazaminopterins dissolved/suspended in pharmaceutically acceptable liquid carrier such as mineral oil or saline or mixed with pharmaceutically acceptable solid carriers such as talc, magnesium stearate, starches, and sugar, and if desired, formed into dosage forms such as tablets, cachets, suppositories and capsules. As used to describe such carriers the term "pharmaceutically acceptable" means that the carrier is nontoxic, generally inert, and does not affect the functionality of the active ingredient(s) adversely. The term carrier includes materials that are sometimes referred to as diluents or vehicles in the pharmaceutical formulation art. The 8,10-dideazaminopterins will mainly be used as aqueous solutions of their sodium salts for administration intraveneously or subcutaneously.

The dose and the dosage regimen will depend upon the neoplasm being treated, the mode of administration, the animal species involved, and the weight of the subject. The dosage will typically range between about 0.1 to 500 mg/day. Treatment will typically be given daily until the neoplasm is in remission. Maintenance treatments during remission will typically be made on an intermittent basis.

The compounds may be administered parenterally (e.g. intravenous, intraarterial, intraperitoneal, intramuscular, intrathecally, subcutaneously) or orally. They are effective in treating leukemias, lymphomas, and solid tumors such as choriocarcinoma, hydatidiform mole, chlorioadenoma destruens, acute lymphocytic leukemia, mycosis fungoides, esteogenic sarcoma, lymphosarcoma, and other non-Hodgkin's lymphomas, breast tumors, bronchogenic carcinomas, tumors of the testis, and Burkitt's lymphoma. They may be administered with other drugs such as chlorambucil. When administered locally to treat a regionalized neoplasm, counteragents such as folinic acid may be given systemically to counteract the systemic activity of the 8,10-dideazaminopterin.

The 8,10-dideazaminopterins are in the class of antineoplastic agents designated antimetabolites. Like methotrexate, they bind tightly to DHFR and prevent the enzyme from activating the conversion of dihydrofolate to tetrahydrofolate. The blockage of this pathway inhibits DNA synthesis thereby inhibiting reproduction of malignant cells. Accordingly, as used herein the term "therapeutically effective amount" means a quantity of one or more of the invention compounds that, when administered to a patient inhibits neoplastic growth. The compounds of the invention thus function similarly to methotexate in therapy but have enhanced antitumor potency and spectrum of activity as compared to methotrexate.

The following examples A and B are comparative examples illustrative of known compounds and examples 1 and 2 illustrate of the claimed compounds.

Example A

Preparation of 10-Methyl-8,10-dideazaminopterin

Step 1. Dimethyl α-Methylhomoterephthalate

A solution of dimethyl homoterephthalate (4.16 g, 20 mmoles) in 15 ml of dry tetrahydrofuran was added dropwise over 15 min to a stirred mixture of potassium hydride (2.5 g of 35% oil suspension, 22 mmole) in 75 ml of dry tetrahydrofuran at 0°C. After 30 min a homogeneous yellow suspension had formed. Methyl iodide (2.79 g, 22 mmole) was then added over a 5 minute period and the resulting mixture stirred for 30 minutes. The mixture was quenched with 2 ml of 50% acetic acid, diluted with 500 ml of water and extracted with ether. The ether extract was washed with water, dried over magnesium sulfate and evaporated to leave an oil. The material was chromatographed on silica gel with elution by ether-hexane (1:9) to afford (71%) of a clear oil; m/e 222 ($M^+$): NMR ($CDCl_3$) 1.50 (3H, d, $CH_3$), 3.65 (3H, s, $OCH_3$), 3.93 (3H, s, $OCH_3$ benzoate).

Step 2. 4-Amino-4-desoxy-10-carbomethoxy-10-methyl-8,10-dideazaminopterate (Dimethyl Ester)

To a stirred mixture of 5.24 g of 22.3% oil suspension of KH (29.2 mmol) and 24 ml of dry DMF at 0° was slowly added 6.48 g (29.2 mmol) of the dimethyl α-methylhomoterephthalate prepared in Step 1. After 30 min the yellow solution was cooled to −40° and 3.6 g (9.2 mmol) of the bromomethyl deazapterin in 48 ml of DMF was added over a 10 min period. The mixture was brought to ambient temperature and stirred for 2 hr. The solvent was removed *in vacuo* and the residue was partitioned between 100 ml of $CHCl_3$ and 25 ml of water. The $CHCl_3$ solution was dried ($MgSO_4$), concentrated *in vacuo* and chromatographed on silica gel with initial elution by $CHCl_3$ to remove unreacted ester, followed by elution of product by $CH_3OH/CHCl_3$ (1:6) to afford 2.85 g (78%) of a yellow solid 4-amino-4-desoxy-10-methyl-10-carbomethoxy-8,10-dideazapteroate, m/e 395 ($M^+$); NMR ($d_6$-DMSO) 1.50 (3H, s, $CH_3$), 3.53 (1H, d, C-9H), 3.59 (3H, s, $OCH_3$), 3.72 (1H, d, C-9H), 3.86 (3H, s, benzoate $OCH_3$), 7.32 (1H, d, C-7H), 7.47 (1H, d, C-8H), 7.54 (2H, d, 3′,5′-H's), 7.96 (2H, d, 2′,6′-H's).

*Anal.* $C_{22}H_{21}O_5O_4 \cdot H_2O$

Calc'd: C, 58.1; H, 5.61; N, 16.9
Found: C, 58.4; H, 5.30; N, 17.0.

Step 3. 4-Amino-4-desoxy-10-methyl-8,10-dideazapteroic Acid

The 10-methyldiester from Step 2 (2.85 g, 7.22 mmol) and 1.06 g (21.7 mmol) of NaCN in 50 ml of DMSO were stirred at 175—180° under $N_2$ for 2.5 hr. The dark mixture was cooled and the solvent removed *in vacuo*. The residue was dissolved in 45 ml of water, the solution filtered and the filtrate acidified with HOAc. The precipitate was collected, washed with water and dried to leave 2.28 g (96%) of 4-amino-4-desoxy-10-methyl-8,10-dideazapteroic acid; m/e 323 ($M^+$); NMR ($d_6$-DMSO) 1.23 (3H, d, $CH_3$), 3.06 (2H, m, 9-$CH_2$), 3.50 (1H, m, 10-H), 7.27 (1H, d, 7-H), 7.39 (3H, m, 8-H, 3′,5′-H's), 7.82 (2H, d, 2′,6′-H's).

Step 4. 10-Methyl-8,10-dideazaminopterin Diethyl Ester

A mixture of 2.38 g (7.3 mmol) of the diamino-10-methylpteroic acid prepared in Step 3, 2.03 ml (14.7 mmol) of triethylamine and 54 ml of dry DMF was treated dropwise with 1.9 ml (14.7 mmol) of isobutyl chloroformate. The mixture was stirred at room temperature for 1 hr and a mixture of 3.52 g (14.7 mmol) of diethyl L-glutamate·HCl, 2.03 ml (14.7 mmol) of triethyl amine and 18 ml of DMF was added. The mixture was stirred at ambient temperature for 24 hr and the solvent was removed *in vacuo*. The residue was partitioned between 50 ml of sat'd. $NaHCO_3$ and 50 ml of $CHCl_3$, followed by two additional $CHCl_3$ extractions. The $CHCl_3$ extract was dried ($MgSO_4$) and evaporated. The crude material was chromatographed on 110 g of silica gel with initial elution by $CHCl_3$, then removal of the product by MeOH/$CHCl_3$ (2.5:97.5) to give 2.01 g (54%) of 10-methyl-8,10-dideazaminopterin diethyl ester as a yellow gum, m/e 508 ($M^+$); NMR ($CDCl_3$) 1.23 (9H, m, 10-$CH_3$, $COOCH_2\mathit{CH_3}$), 2.4 (4H, m, $\mathit{CH_2CH_2}COOCH_2CH_3$), 3.1—3.5 (3H, m, 10-H, 9-$CH_2$), 4.3 (4H, q, $COO\mathit{CH_2}CH_3$), 4.8 (1H, d, CONH*CH*, 7.2—7.4 (4H, m, 3′-H, 5′H, 7-H, 8-H) 7.8 (2H, d, 2′-H, 6′-H).

Step 5. 10-Methyl-8,10-dideazaminopterin

A solution of 0.43 g of the 10-methyl-8,10-dideazaminopterin diethyl ester prepared in Step 4 in 6.5 ml of 2-methoxyethanol was treated with 4.3 ml of 1N NaOH. The solution was maintained at ambient temperature for 3 hr and diluted with 35 ml of $H_2O$. The solution was acidified with HOAc to precipitate the product. After refrigeration for 24 hr the precipitate was collected, washed with $H_2O$ and dried to leave 0.265 g (70%) of 10-methyl-8,10-dideazaminopterin also as a pale yellow microcrystalline solid; m/e as tetratrimethylsilyl derivative, 740, ($M^+$).

Example B

Preparation of 10-Propyl-8,10-dideazaminopterin

Step 1. Dimethyl α-Propylhomoterephthalate

Dimethyl homoterephthalate was alkylated as in Example 1, Step 1 but with propyl bromide to afford the α-propyl product in 74% yield as an oil, m/e 250 ($M^+$); NMR ($CDCl_3$) 0.91 (3H, 5, $CH_3$), 1.26 (2H, m, —$\mathit{CH_2}CH_3$), 1.75—2.05 (2H, m, —$\mathit{CH_2}C_2H_5$), 3.62 (1H, t, *CH*—$C_3H_7$), 3.66 (3H, s, $OHC_3$) 3.91 (3H, s, benzoate $OCH_3$).

Step 2. 4-Amino-4-desoxy-10-carbomethoxy-10-propyl-8,10-dideazapterioc Acid Dimethyl Ester

The procedure described in Example 1, Step 2 was carried out except that dimethyl α-propylhomoterephthalate was reacted with the bromomethyl compound. The product diester was obtained as a yellow solid in 45% yield; m/e 423 ($M^+$); NMR ($d_6$-DMSO) 0.72 (3H, t, $CH_3$), 1.05 (2H, m, —$\mathit{CH_2}CH_3$), 1.92 (2H, m, —$\mathit{CH_2}$C—COOMe) 3.57 (3H, s, $COOCH_3$), 3.63 (2H, m, 9-H's), 3.85 (3H, s, benzoate $COOCH_3$).

Step 3. 4-Amino-4-desoxy-10-propyl-8,10-dideazaminopteroic Acid

The dimethyl ester of Step 2, this Example, was decarboxylated as in Example 1, Step 3, to give the 10-propyl product as a yellow solid in 81% yield, m/e 351 ($M^+$); NMR ($d^6$-DMSO) 0.82 (3H, t, $CH_3$), 1.15 (2H, m, $\mathit{CH_2}$, $CH_3$), 1.63 (2H, m, —$\mathit{CH_2}$CH), 3.1 (2H, m, 9-$CH_2$), 3.5 (1H, m, 10-H), 7.20 (1H, d, 7-H), 7.31 (1H, d, 8-H), 7.36 (2H, d, 3′,5′-H's), 7.79 (2H, d, 2′,6′-H's).

Steps 4 and 5. 10-Propyl-8,10-dideazaminopterin

The pteroic acid was condensed with diethyl L-glutamate in the manner described in Example 1, Step 4. The crude ester was obtained in 38% yield; TLC ($CHCl_3$-MeOH, 9:1) $R_f$ 0.40; m/e 536 ($M^+$). The ester was saponified as in Example 1, Step 5, to give the product 10-propyl-8,10-dideazaminopterin in 53% yield, m/e for the tetratrimethylsilyl derivative, 768 ($M^+$).

Example 1

Preparation of 10-ethyl-8,10-dideazaminopterin

Step 1. Dimethyl α-ethyl Homoterephthalate

Using the procedure of Step 1, Example 1, but substituting for methyl iodide, ethyl iodide, dimethyl α-ethyl homoterephthalate was obtained in 72% yield, m/e 236; NMR ($CDCl_3$) 0.9 (3H, t, $CH_3$), 2.0 (2H, m, $CH_2CH_3$), 3.5 (1H, m, $CHC_2H_5$) 3.70 (3H, s, $OCH_3$), 3.90 (3H, s, benzoate $OCH_3$).

Step 2. 4-Amino-4-desoxy-10-carbomethoxy-10-ethyl-8,10-dideazapteroic Acid Dimethyl Ester

Using the procedure of Step 2 of Example 1, and the product of Step 1 of this Example, 4-amino-4-desoxy-10-carbomethoxy-10-ethyl-8,10-dideazapteroic acid dimethyl ester was obtained in 74% yield, m/e 409 ($M^+$); NMR ($d_6$-DMSO) 0.73 (3H, t, $CH_3$), 2.20 (2H, m, $CH_2CH_3$), 3.67 (5H, m, 9-$CH_2$, $COOCH_3$), 3.90 (3H, s, benzoate $OCH_3$).

Step 3. 4-Amino-4-desoxy-10-ethyl-8,10-dideazaminopteric Acid

Using the procedure of Step 3 of Example 1, and the product of Step 2 of this Example, 4-amino-4-desoxy-10-ethyl-8,10-dideazaminopteric acid was obtained and analytical samples obtained by recrystallization from DMSO.

Calcd for $C_{18}H_{19}N_5O_2$. C, 64.1; H, 5.68; N; 20.8;
Found: C, 63.9; H, 5.78; N, 20.5;

UV abs: 238 nm ε = 35,374; 343 nm, ε = 5,134.

Step 4. 10-Ethyl-8,10-dideazaminopterin Diethyl Ester

Using the procedure of Step 4, Example 1, and the product of Step 3 of this Example, 10-ethyl-8,10-dideazaminopterin diethyl ester was obtained in 34% yield, m/e 522 ($M^+$).

Step 5. 10-Ethyl-8,10-dideazaminopterin

The 10-ethyl-8,10-dideazaminopterin diethyl ester of the previous step was hydrolyzed under the conditions of Step 5, Example 1 to give 10-ethyl-8,10-dideazaminopterin as $C_{23}H_{26}N_6O_5 \cdot 1.25\ H_2O$.

Calcd: C, 56.5; H, 5.87; N, 17.2;
Found: C, 56.6; H, 5.91; N, 17.0;

UV absorption max: 238 nm, ε = 37,652; 343 nm, ε = 5,298.

Example 2

The effectiveness of the invention compounds as antineoplastic agents may be demonstrated by subjecting them to the L-1210 murine leukemia assay. This is a standard assay that is used to evaluate the efficacy of compounds as antineoplastic agents for treating humans. The assay is described by D. J. Hitchinson, D. C. Robinson, D. Martin, O. L. Ittenson and Dillenberg, *Journal Cancer Research,* (1962) *22*:57—72. It is a procedure for evaluating a compound for increase in median life span in treated animals and unteated controls both infected with L-1210 leukemia cells. The test hydroxide, adjusted to pH 7, and the solution diluted to 10 ml with distilled water. Mice are injected once per day every other day for a total of 5 injections starting one day after transplantation of $10^6$ tumor cells/mouse. The results of this assay with several, 8,10-dideazaminopterin analogs were as follows:

| Compound | $LD_{10}$ Dose:Mg/Kg | ILS% | Cells Remaining |
|---|---|---|---|
| 8,10-dideazaminopterin | 0.75 | +189 | 4000 |
| 10-methyl-8,10-dideazaminopterin | 0.6 | +203 | 1000 |
| 10-ethyl-8,10-dideazaminopterin | 1.5 | +235 | 100 |
| 10-n-propyl-8,10-dideazaminopterin | 6.0 | +176 | 10,000 |
| Methotrexate | 15 | +187 | |
| Control | — | — | |

It is apparent from the above results that the life spans of the mice were increased considerably by administration of the invention compound and that this compound has surprising activity when compared to the non-alkylated and n-propyl or methyl analogs. The % ILS data represent comparisons of log values, and thus differences in the % ILS which appear minor mask large differences in cytotoxic activity. Thus, as shown, the ethyl derivative is 40 times as effective as the unsubstituted compound and 100 times as effective as the n-propyl derivative in terms of reducing the number of neoplastic cells; i.e., e.g., the number of cells remaining after 10-ethyl analog treatment is only 1/40 and 1/100 of that remaining from treatment with the above analogs, respectively. The assay and these conclusions are described by Schabel, F. M., *Symposium on Proliferation and Spread of Neoplastic Cells,* M. D. Anderson, Tumor Institute, Houston, Texas (1968), p. 379 ff.

**Claims**

1. A compound of the formula

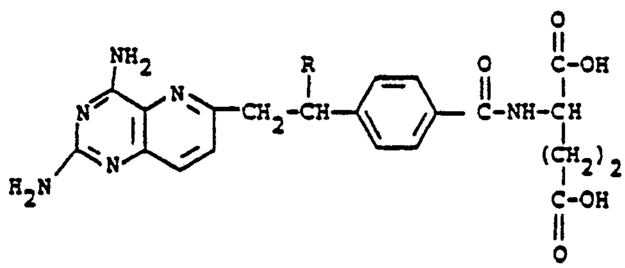

where R is ethyl and pharmaceutically acceptable salts thereof.

2. The compound of claim 1 wherein said pharmaceutically acceptable salts are sodium salts.

3. 10-Ethyl-8,10-dideazaminopterin.

4. An antineoplastic composition comprising a therapeutically effective amount of the compound of claim 1 or a salt thereof combined with a pharmaceutically acceptable carrier.

**Patentansprüche**

1. Verbindung der Formel

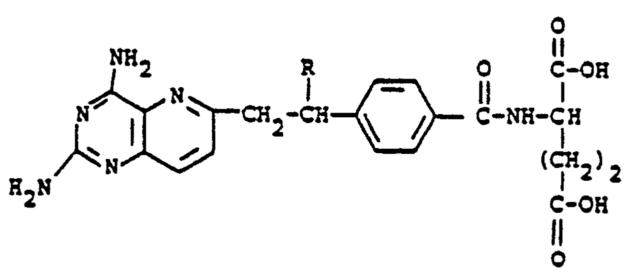

worin R Ethyl ist, und pharmazeutisch geeignete Salze davon.

2. Verbindung nach Anspruch 1, wonach die pharmazeutisch geeigneten Salze Natriumsalze sind.

3. 10-Ethyl-8,10-dideazaminopterin.

4. Antineoplastiche Zusammensetzung mit einem Gehalt an einer therapeutisch wirksamen Menge der Verbindung nach Anspruch 1 oder an einem Salz davon, kombiniert mit einem pharmazeutisch geeigneten Träger.

**Revendications**

1. Composé de formule

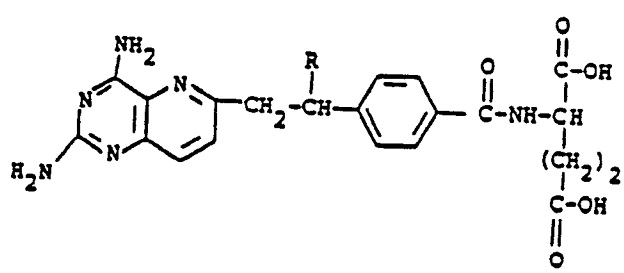

dans laquelle R représente un groupe éthyle, et ses sels pharmaceutiquement acceptables.

2. Composé suivant la revendication 1, dans lequel les sels pharmaceutiquement acceptables sont des sels de sodium.

3. 10-éthyl-8,10-didéazaminoptérine.

4. Composition antinéoplastique comprenant une quantité à effet thérapeutique du composé suivant la revendication 1, ou d'un de ses sels, associée à un support pharmaceutiquement acceptable.